(19) Europäisches Patentamt
European Patent Office
Office européen des brevets

(11) Publication number: **0 306 575 B1**

(12) **EUROPEAN PATENT SPECIFICATION**

(45) Date of publication of patent specification: **29.07.92**

(51) Int. Cl.[5]: **A61K 31/485**, //C07D489/08

(21) Application number: **87307999.0**

(22) Date of filing: **10.09.87**

(54) **Ouaternary derivatives of noroxymorphone which relieve nausea and emesis.**

(43) Date of publication of application:
**15.03.89 Bulletin 89/11**

(45) Publication of the grant of the patent:
**29.07.92 Bulletin 92/31**

(84) Designated Contracting States:
**AT BE CH DE ES FR GB GR IT LI LU NL SE**

(56) References cited:
**WO-A-83/03197**
**GB-A- 1 202 148**
**US-A- 4 176 186**
**US-A- 4 466 968**

**CHEMICAL ABSTSTRATS, vol. 100, no. 3, 16th January 1984, page 42, abstract no. 17503x; M. DRAGONETTI et al.: "Methyllevallorphan iodide (SR 58002), a potent narcotic antagonist with peripheral selectivity superior to that of other quaternary compounds"; & LIFE SCI. 1983, 33(Suppl. 1), 477-480**

**CHEMICAL ABSTRACT, vol. 96, no. 21. 24th May 1982, page 60, abstract no. 174238f; J.C. KEITH, JR. et al.: "Failure of naloxone to prevent the emetic activity of apomorphine in dogs"; & J. VET. PHARMACOL. THER. 1981, 4(4), 315-316**

**CHEMICAL ABSTRACTS, vol. 101, no. 25, 17th December 1984, page 19, abstract no. 222104e; M.A. IORIO et al.: "Narcotic agonist/antagonist properties of quaternary diastereoisomers derived from oxymorphone and naloxone", & EUR.J.MED.CHEM.-CHIM.THER. 1984, 19(4), 301-303**

(73) Proprietor: **THE UNIVERSITY OF CHICAGO**
**947 East 58th Street**
**Chicago, Illinois 60657(US)**

(72) Inventor: **Goldberg, Leon I.**
**5000 Cornell Avenue**
**Chicago Illinois 60615(US)**

(74) Representative: **Holdcroft, James Gerald, Dr. et al**
**Graham Watt & Co., Riverhead**
**Sevenoaks, Kent TN13 2BN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 306 575 B1

## Description

The administration of therapeutic doses of morphine and other clinically useful narcotic analgesics is often accompanied by unpleasant side effects on the gastro-intestinal system. For instance, morphine and related opiates such as meperidine and methadone may retard intestinal mobility by causing contractions of the small bowel circular smooth muscle.

Morphine and related narcotics may also induce nausea and increased mobility of the gastrointestinal tract resulting in emesis or vomiting. These side effects are caused by direct stimulation of the chemoreceptor trigger zone for emesis in the area postrema of the medulla. (Goodman and Gilman, The Pharmacological Basis of Therapeutics, p. 502 [6th ed. 1980],) Studies have shown that morphine and other narcotics cause emesis in dogs. For example, Wang and Glaviano, JPET 111:329-334 (9143), reported that administration of 0.5 mg/kg of morphine intravenously to 12 dogs resulted in emesis in 9 dogs within an average of 2.4 minutes. (Mg/kg refers to milligrams of morphine per kilograms of body weight.) When 1.0 mg/kg of morphine was administered intramuscularly to 13 dogs, 12 of them vomited within an average time of 3.5 minutes.

U. S. Patent No. 4,176,186 of Goldberg et al disclosed treatment of intestinal immobility associated with the use of narcotic analgesics through the administration of quaternary derivatives of noroxymorphone. It has now been discovered that the same compounds are also useful for the treatment, both prophylactic and therapeutic, of the nausea and vomiting associated with the administration of these drugs.

According to the invention, therefore, nausea and vomiting by warm-blooded animals receiving morphine and related opiates, meperidine, methadone, may be prevented or relieved by the administration of methylnaltrexone or other quaternary derivatives of noroxymorphone represented by the formula:

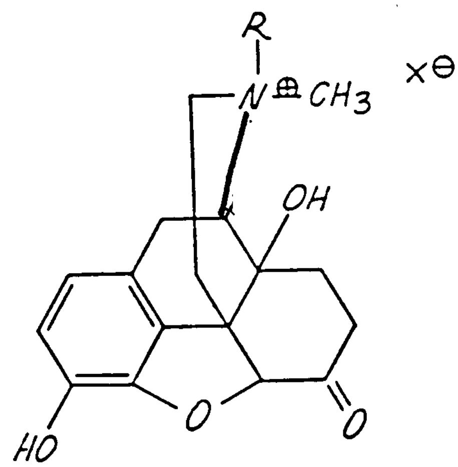

wherein

R is allyl or a related radical such as chloroallyl, cyclopropyl-methyl or propargyl, and

X is the anion of an acid, especially a chloride, bromide, iodide or methylsulfate anion.

These compounds are administered to the animal either prior to or simultaneously with the administration of the narcotic analgesic. They may be administered either enterally or parenterally. There has not been observed any interference with the analgesic activity of the opiates.

As used herein, unless the sense of the usage indicates otherwise, the term "morphine" refers to any narcotic analgesic.

This invention relates to the use of quaternary derivatives of noroxymorphone for the manufacture of a medicament for preventing or relieving nausea and vomiting associated with the administration of morphine to warm-blooded animals. The useful compounds are represented by the formula:

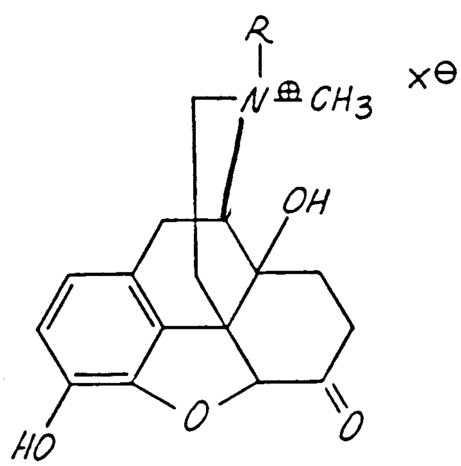

wherein

R is allyl or a related radical such as chloroallyl, cyclopropyl-methyl or propargyl, and

X is the anion of an acid, especially a chloride, bromide, iodide or methylsulfate anion.

The compounds are synthesized as described in United States Patent No. 4,176,186. A particularly preferred noroxymorphone derivative is methylnaltrexone, but other compounds represented by the above formula are also suitable.

Methylnaltrexone or other noroxymorphone derivatives may be administered to the patient either enterally or parenterally. However, a preferred method of administration is by injection. Nausea and emesis may follow after even a single does of morphine, unlike intestinal immobility which is usually the effect of chronic repeated usage of the drug. Consequently, it is contemplated that the patient will be given an injection of methylnaltrexone prior to surgery or other occasion when morphine is used to treat acute pain.

As illustrated by the following Controls and Examples, our studies show that methylnaltrexone inhibits emesis when administered either together with the morphine or before the morphine is administered. It is thought that methylnaltrexone or other quaternary noroxymorphone derivatives may be administered up to two hours before the administration of morphine, but that period may be variable. In our studies, methylnaltrexone was administered intramuscularly by means of a syringe. Methylnaltrexone may also be administered enterally or parenterally by other means. It has been found to be effective in dosages in the range of 0.05 mg/kg to 1.0 mg/kg for each 1 mg/kg of administered morphine. It was found effective when administered in the same syringe as morphine and also when administered up to one hour before the administration of morphine.

The effect of methylnaltrexone in reversing the emetic effects of morphine is illustrated herein. The unit of mg/kg refers to milligrams of substance administered per kilograms of body weight.

## CONTROL 1 AND EXAMPLE 1

One mg/kg of morphine was administered intramuscularly to five dogs. Four dogs vomited. In each instance, vomiting occurred within four minutes. On a different day the same dose of morphine was administered intramuscularly to the same five dogs in the same syringe with 1 mg/kg of methylnaltrexone. None of the dogs vomited.

## CONTROL 2 AND EXAMPLE 2

Six dogs were given intramuscular doses of 1 mg/kg of morphine. All six dogs vomited. On an additional day the same dose of morphine was combined with 0.5 mg/kg of methylnaltrexone and administered in the same syringe to the same dogs. None of the dogs vomited.

## CONTROL 3 AND EXAMPLE 3

One mg/kg of morphine was administered intramuscularly to three dogs. All three dogs vomited. On an additional day the morphine was combined with 0.25 mg/kg of methylnaltrexone and administered in the same syringe. None of the dogs vomited.

## CONTROL 4 AND EXAMPLE 4

Methylnaltrexone was administered to two dogs prior to the administration of 1 mg/kg morphine. In one dog, 0.5 mg/kg of methylnaltrexone was administered intramuscularly 15 minutes before the morphine. No vomiting occurred. In the second dog, the same dose of methylnaltrexone was administered 30 minutes before the administration of morphine. No vomiting occurred.

## CONTROL 5 AND EXAMPLE 5

0.05 mg/kg methylnaltrexone was administered intravenously to four dogs one minute prior to the administration of 1.0 mg/kg morphine. No vomiting occurred in any of the dogs. On a different day, the same animals were given 1.0 mg/kg morphine without the administration of methylnaltrexone. All four dogs vomited.

The administration of methylnaltrexone alone was found to produce no noticeable effects in the animals. Previous studies with larger doses of methylnaltrexone have demonstrated that unlike the non-quaternary naltrexone, methylnaltrexone does not precipitate withdrawal systems in morphine-tolerant dogs. Russell et al., Eur. J. Pharmacol. 78:255-261 (1982). Methylnaltrexone has not been found to interfere with the analgesic activity of morphine or narcotics.

## Claims

**1.** Use of a compound of the formula:

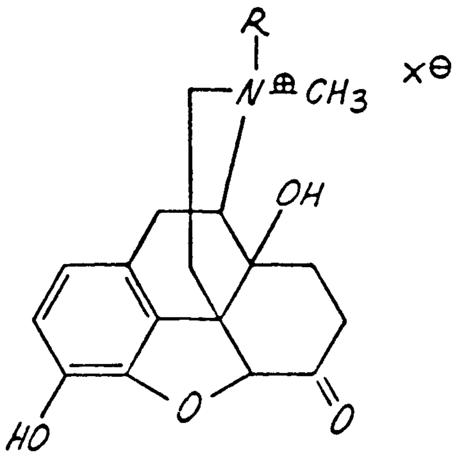

wherein

R is allyl or a related radical e.g. chloroallyl, cyclopropyl-methyl or propargyl, and

X is the anion of an acid, e.g. a chloride, bromide, iodide or methylsulfate anion for the manufacture of a medicament for preventing or relieving nausea and emesis associated with the use of narcotic analgesics in warm-blooded animals.

**2.** Use according to claim 1, wherein the medicament is produced in unit dosage form for administration in an amount between 0.05 mg/kg and 1.0 mg/kg.

**3.** Use according to claim 1 or claim 2, wherein the medicament is produced in a form for administration to the animal enterally, or by injection.

**4.** Use according to claim 1 or claim 2, wherein the medicament is produced in a form for administration to the animal parenterally.

**5.** Use according to any one of claims 1 to 4, wherein the compound is admixed with the narcotic analgesic for the manufacture of said medicament.

**6.** Use according to any one of claims 1 to 5, wherein the compound is methylnaltrexone.

**7.** Use of methylnaltrexone for the manufacture of a medicament for preventing or relieving nausea and emesis associated with the use of narcotic analgesics in warm-blooded animals, for administering e.g. parenterally to an animal prone to exhibit nausea or emesis on administration of narcotic analgesics.

**Revendications**

**1.** Utilisation d'un composé de formule

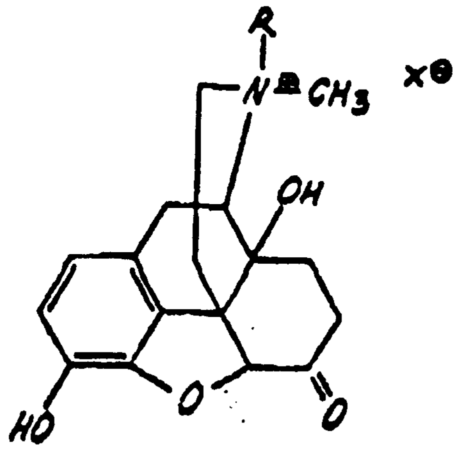

dans laquelle

R est un radical allyle ou apparenté, par exemple chloro-allyle, cyclopropylméthyle ou propargyle,

X est l'anion d'un acide, par exemple un anion chlorure, bromure, iodure ou méthylsulfate,

pour la préparation d'un médicament de prévention ou de soulagement des nausées et vomissements associés à l'emploi d'analgésiques narcotiques chez des animaux à sang chaud.

**2.** Utilisation selon la revendication 1, dans laquelle le médicament est préparé sous forme d'unités posologiques pour leur administration à une dose comprise entre 0,05 mg/kg et 1,0 mg/Kg.

**3.** Utilisation selon la revendication 1 ou 2, dans laquelle le médicament est préparé sous une forme propre à l'administration à l'animal par voie digestive ou par ingestion.

**4.** Utilisation selon la revendication 1 ou 2, dans laquelle le médicament est préparé sous une forme propre à l'administration parentérale à l'animal.

**5.** Utilisation selon l'une quelconque des revendications 1 à 4, dans laquelle le composé est mélangé à l'analgéstque narcotique pour la préparation dudit médicament.

**6.** Utilisation selon l'une quelconque des revendications 1 à 5, dans laquelle le composé est la méthylnaltrexone.

7. Utilisation de méthylnaltrexone pour la préparation d'un médicament de prévention ou de soulagement des nausées et vomissements associés à l'emploi d'analgésiques narcotiques chez des animaux à sang chaud, propre à être administré, par exemple par voie parentérale, à un animal sujet aux nausées ou aux vomissements en cas d'administration d'analgésiques narcotiques.

**Patentansprüche**

1. Verwendung einer Verbindung der Formel

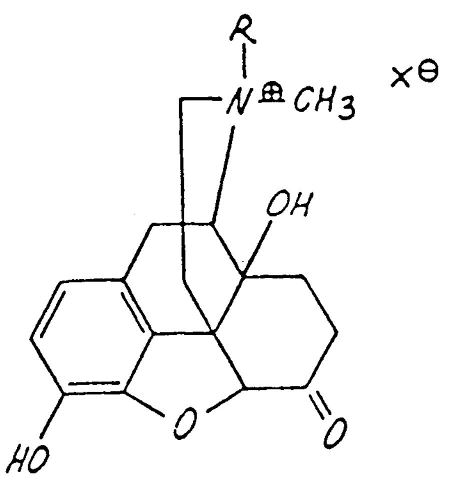

wobei
R Allyl oder ein verwandtes Radikal z.B. Chlorallyl, Cyclopropyl-methyl oder Propargyl ist und
X das Anion einer Säure ist, z.B. ein Chlorid, Bromid, Iodid oder Methylsulfatanion, zur Herstellung eines Medikaments zur Verhinderung oder Linderung von Nausea und Emesis im Zusammenhang mit der Verwendung von Schmerzbetäubungsmitteln bei Warmblütern.

2. Verwendung nach Anspruch 1, bei der das Medikament in einer Einheitsdosisform zur Verabreichung in einer Menge zwischen 0,05 mg/kg und 1,0 mg/kg hergestellt wird.

3. Verwendung nach Anspruch 1 oder Anspruch 2, bei der das Medikament in einer Form zur Verabreichung an das tierische Lebewesen auf enteralem Wege oder durch Injektion hergestellt wird.

4. Verwendung nach Anspruch 1 oder Anspruch 2, bei der das Medikament in einer Form zur Verabreichung an das tierische Lebewesen auf parenteralem Wege hergestellt wird.

5. Verwendung nach einem der Ansprüche 1 bis 4, bei der die Verbindung dem Schmerzbetäubungsmittel zur Herstellung des Medikaments beigemischt wird.

6. Verwendung nach einem der Ansprüche 1 bis 5, bei der die Verbindung Methylnaltrexon ist.

7. Verwendung von Methylnaltrexon zur Herstellung eines Medikaments zur Verhinderung oder Linderung von Nausea und Emesis im Zusammenhang mit der Verwendung von Schmerzbetäubungsmitteln bei Warmblütern zur Verabreichung z.B. auf parenteralem Wege an ein tierisches Lebewesen, das dazu neigt, bei Verabreichung von Schmerzbetäubungsmitteln Nausea oder Emesis zu zeigen.